# EUROPÄISCHE PATENTSCHRIFT

(11) **EP 2 401 009 B1**
(45) Veröffentlichungstag und Bekanntmachung des Hinweises auf die Patenterteilung: **28.11.2012**
(21) Anmeldenummer: 09779097.6
(22) Anmeldetag: 26.02.2009
(51) Int. Cl.: A61M 5/24

(54) **PRODUKTBEHÄLTNISHALTER FÜR EINE INJEKTIONSVORRICHTUNG UND ZUR AUFNAHME EINES PRODUKTBEHÄLTNISSES**
PRODUCT CONTAINER HOLDER FOR AN INJECTION DEVICE AND FOR RECEIVING A PRODUCT CONTAINER
SUPPORT DE RÉSERVOIR À PRODUIT POUR UN DISPOSITIF D'INJECTION ET POUR LA RÉCEPTION D'UN RÉSERVOIR À PRODUIT

(43) Veröffentlichungstag der Anmeldung: 04.01.2012
(73) Patentinhaber: Tecpharma Licensing AG, 3401 Burgdorf (CH)
(72) Erfinder: BOLLENBACH, Markus, CH-3014 Bern (CH); STREIT, Ursina, 3222 Schönbühl (CH); KÜNZLI, Daniel, CH-4513 Langendorf (CH); KAUFMANN, Nadine, CH-3400 Burgdorf (CH); MOSER, Ulrich, CH-3412 Heimiswil (CH)
(86) Internationale Anmeldenummer: PCT/EP2009/052323
(87) Internationale Veröffentlichungsnummer: WO 2010/097116

(56) Entgegenhaltungen:
- WO-A-00/24441
- WO-A-2005/097252
- DE-A1-102007 013 836
- GB-A- 2 434 317
- US-A- 3 144 178
- US-A- 5 002 537

## Beschreibung

Die Erfindung betrifft einen Produktbehältnishalter für eine Injektionsvorrichtung zur Verabreichung eines vorzugsweise flüssigen Produkts, insbesondere Medikaments. Die Erfindung betrifft ferner eine Baugruppe umfassend ein Produktbehältnis und einen Produktbehältnishalter, sowie ein Verfahren zur Montage einer Injektionsvorrichtung, insbesondere einer Autoinjektionsvorrichtung oder eines Autoinjektors.

Aus dem Stand der Technik sind Injektionsgeräte, insbesondere so genannte Autoinjektoren bekannt. Bei Autoinjektoren wird nach deren Auslösung automatisch Produkt verabreicht, d.h: eine Nadel automatisch in das Gewebe eines Patienten eingestochen und anschließend das Produkt automatisch ausgeschüttet. Bei manchen Autoinjektoren wird sogar die Nadel automatisch in den Autoinjektor zurückgezogen, nachdem die Produktausschüttung beendet ist. Bei anderen Autoinjektoren wird die Nadel manuell aus der Einstichstelle herausgezogen.

Aus der GB2434317, US5002537, WO2005097252, US3144178 sind Injektionsvorrichtungen, insbesondere Autoinjektoren bekannt, die einen Spritzenhalter aufweisen. Die Schrift GB2434317 zeigt einen Spritzenhalter welcher die Spritze am distalen Ende anstatt am proximalen Flange 90 sichert. Es wird ein Spritzenhalter beschrieben welcher die Spritze an der vorderen Schulter mittels einer Schulterauflage abstützt und durch Kraftschluss hält Diese Schulterauflage weist flexible Finger 108 auf, welche sich radial öffnen können und durch elastische Mittel (spring retainer 111) in ihrer Normalposition gehalten werden.

Bei Autoinjektoren ist ein Produktbehältnis mit dem zu verabreichenden Produkt oftmals in einen Produktbehältnishalter aufgenommen. Die aufgenommenen Produktbehältnisse können so genannte Standardspritzen sein, die oftmals aus Glas gefertigt sind. Um aufwändige Zulassungsverfahren für Produktbehältnisse zu vermeiden, werden bevorzugt Standardspritzen, die bereits eine Zulassung haben, in Injektionsvorrichtungen oder Autoinjektoren verbaut. Standardspritzen können an ihrem distalen, d.h. der Nadel gegenüber liegenden Ende einen Flansch aufweisen, der auch als Fingerflansch bezeichnet wird, da er bei der manuellen Verabreichung der Spritze ein Widerlager für den Mittel- und den Zeigefinger bildet. Aus dem Stand der Technik ist es bekannt, den Flansch gleichzeitig zur Befestigung des Produktbehältnisses an einem Produktbehältnishalter zu verwenden, wie es beispielsweise in der DE 10 2007 013 836 A1 beschrieben wird. Beim Vorschieben des Produktbehältnisses zum Einstechen der Nadel und zum Ausschütten des Produkts werden durchaus hohe Kräfte bzw. Impulse auf den Spritzenflansch ausgeübt. Dies kann unter Umständen dazu führen, dass der Spritzenflansch nach dem Auslösen der Injektionsvorrichtung bricht, wodurch eine ordnungsgemäße Produktverabreichung nicht mehr möglich ist.

Da Injektionsvorrichtungen, insbesondere auch Autoinjektoren in hohen Stückzahlen gefertigt werden, werden sie maschinell zusammengesetzt. An die Teile der Injektionsvorrichtung werden daher neben hohen Sicherheitsanforderungen auch noch Anforderungen bezüglich der Handhabbarkeit bzw. der maschinellen Verarbeitbarkeit, insbesondere Montage, gestellt.

Es ist eine Aufgabe der Erfindung, einen Produktbehältnishalter zur Aufnahme eines Produktbehältnisses bereit zu stellen, der das Produktbehältnis vor Beschädigungen bei der Verabreichung schützt und einfach montierbar ist. Es ist ferner eine Aufgabe der Erfindung, eine Baugruppe aus Produktbehältnishalter und Produktbehältnis anzugeben, bei der das Produktbehältnis vor Beschädigungen bei der Verabreichung geschützt wird und die einfach montierbar ist. Eine weitere Aufgabe der Erfindung ist es, ein Verfahren zur Montage einer Injektionsvorrichtung anzugeben, bei der das Produktbehältnis auf einfache Weise in dem Produktbehältnishalter montierbar ist.

Diese Aufgaben werden gelöst durch die unabhängigen Ansprüche. Vorteilhafte Weiterentwicklungen ergeben sich aus den abhängigen Ansprüchen und aus der Beschreibung.

Die Erfindung geht aus von einem Produktbehältnishalter zur Aufnahme eines Produktbehältnisses, wobei der Produktbehältnishalter eine separat handhabbare Einheit und in eine Injektionsvorrichtung zur Verabreichung eines Produkts einsetzbar ist. Wenn das Produktbehältnis in den Produktbehältnishalter eingesetzt ist, umgibt der Produktbehältnishalter vorzugsweise das Produktbehältnis zumindest zum Teil vorzugsweise zum größten Teil oder vollständig. Der Produktbehältnishalter bildet einen festen Sitz für das Produktbehältnis. Somit kann das Produktbehältnis zusammen mit dem Produktbehältnishalter als Einheit in der Injektionsvorrichtung verschoben werden, um zum Beispiel eine Einstechbewegung einer an dem Produktbehältnis angeordneten Nadel auszuführen. Dabei ist das Produktbehältnis relativ zum Produktbehältnishalter feststehend, während sich der Produktbehältnishalter relativ zu der Injektionsvorrichtung, wie zum Beispiel zu einem Gehäuse bewegen kann.

Das Produktbehältnis kann individuell angepasst sein, vorzugsweise ist es jedoch ein Standardproduktbehältnis. Beispielsweise kann es sich bei dem Produktbehältnis um eine Ampulle oder Karpule handeln, an deren vorderen Ende eine Nadel anbringbar und deren vorderes Ende mit einem durchstechbaren Septum verschlossen ist. Die Nadel kann z. B. mit einer Lurkupplung oder mit einem anderen geeigneten Verbindungsmittel am Produktbehältnis z.B. lösbar befestigt werden.

Bevorzugt ist das Produktbehältnis eine so genannte Spritze, die wie eine Karpule oder Ampulle auch, einen Aufbewahrungsabschnitt für das Produkt aufweist. Der Aufbewahrungsabschnitt ist bevorzugt zylindrisch. Der Aufbewahrungsabschnitt ist an seinem distalen Ende verjüngt, so dass er eine Schulter bildet, und bis auf eine kleine Öffnung zur Ausschüttung des Produkts verschlossen. Proximal des distalen Verschlusses des Aufbewahrungsabschnitts befindet sich das Produkt, das distal von einem dichtend an dem zylindrischen Aufbewahrungsabschnitt anliegenden und verschiebbaren Kolben abgeschlossen wird. Der Kolben ist zur Ausschüttung des Produkts in distale Richtung verschiebbar, wodurch das Produkt aus dem Aufbewahrungsabschnitt verdrängt wird. An die Spritze kann eine Nadel lösbar angebracht, wie zum Beispiel angeschraubt werden. Bevorzugt ist die Nadel jedoch fest, d.h. unlösbar mit der Spritze verbunden. Die Nadel ist fluidisch mit dem Produkt verbunden, so dass beim Verschieben des Kolbens in distale Richtung das Produkt durch die Nadel abgegeben wird. Das Produktbehältnis, insbesondere die Spritze weist an ihrem proximalen Ende einen Flansch auf, der auch als Fingerflansch bezeichnet werden kann. Wenn das Produktbehältnis in den Produktbehältnishalter vollständig eingesetzt ist, kann der Flansch in einem Anschlag mit dem Produktbehältnishalter sein. Allerdings wird bevorzugt, dass das Produktbehältnis, wenn es vollständig in den Produktbehältnishalter eingesetzt ist, nicht in einen Anschlag mit dem Produktbehälmishalter kommt, um eine Beschädigung aufgrund von Einstech- und Ausschüttkräften, die bei der Verabreichung auf das Produktbehältnis wirken können, sicher zu vermeiden.

Der Produktbehältnishalter kann als separat handhabbare Einheit bezeichnet werden, da er sowohl innerhalb als auch außerhalb der Injektionsvorrichtung mit seinen Teilen und Einrichtungen als Einheit gehandhabt werden kann. Die Teile des Produktbehältnishalters können in und außerhalb der Injektionsvorrichtung selbsttätig zusammenhalten. Der Produktbehältnishalter kann ein- oder mehrteilig gebildet sein, vorzugsweise ist er zumindest im Ursprungszustand, insbesondere vor einer Montage, einteilig. Im Endzustand, insbesondere nach der vollständigen Montage, kann er zwei- oder mehrteilig sein, ohne dass ein Teil hinzugefügt wird. Dadurch, dass der Produktbehältnishalter eine separat handhabbare Einheit bildet, ist dessen Montage auf sehr einfache Weise möglich.

Bei der Injektionsvorrichtung zur Verabreichung des Produkts handelt es sich in einer bevorzugten Ausführungsform, um einen Autoinjektor oder eine Autoinjektionsvorrichtung, wie sie z.B. einleitend erwähnt ist. Bevorzugt wird eine Nadel nach dem Auslösen der Injektionsvorrichtung automatisch eingestochen und das Produkt über die Nadel automatisch abgegeben. Optional kann die Nadel automatisch nach dem Einstechen zurückgezogen werden. Eine derartige Vorrichtung wird in der DE 10 2007 013 836 A1 beschrieben, auf die hinsichtlich ihrer Einstech-, Ausschütt- und Rückzugsmechanik sowie ihres Bewegungsablaufs Bezug genommen wird.

Der Produktbehältnishalter weist eine Längsachse auf, der in aller Regel der Längsachse des Produktbehältnisses und der Nadel entspricht. Ferner kann die Längsachse in bevorzugten Ausführungen der Längsachse der Injektionsvorrichtung entsprechen.

Der Produktbehältnishalter umfasst ein quer zu der Längsachse des Produktbehältnishalters bewegbar angeordnetes Eingriffsglied für das Produktbehältnis. Mit dem Eingriffsglied ist die Bewegung des Produktbehältnisses relativ zu dem Produktbehältnishalter in distale Richtung blockierbar. Die Bewegung des Eingriffsglieds quer zur Längsachse kann eine lineare Bewegung oder eine Schwenkbewegung sein. Die Wirkungsweise des Eingriffsglieds kann auf einem Fonn- oder/und Kraft-, insbesondere Reibschluss basieren. Zweckmäßigerweise kann das Eingriffsglied in einen Eingriff mit dem Produktbehältnis bewegt werden. Das Eingriffsglied kann beispielsweise formschlüssig in eine auf der Außenseite des Produktbehältnisses gebildete Schulter, die distal des Aufbewahrungsabschnitts gebildet wird, eingreifen. Dabei bildet das Eingriffsglied eine Reaktionsfläche, welche eine in distale Richtung auf das Produktbehältnis wirkende Kraft aufnehmen und in den Produktbehältnishalter ableiten kann. Das Eingriffsglied bildet in diesem Fall einen festen Sitz für das Produktbehältnis, so dass es an einer Bewegung relativ zum Produktbehältnishalter in distale Richtung gehindert ist. Ein formschlüssiges Eingriffsglied kann z. B. als nach innen ragender Nocken ausgebildet sein.

In einer anderen Ausführung kann das Eingriffsglied an den Aufbewahrungsabschnitt z. B. seitlich oder an der zylindrischen Außenfläche des Aufbewahrungsabschnitts angreifen und das Produktbehältnis einklemmen. Ein solcher Eingriff kann als kraftschlüssiger Eingriff bezeichnet werden, da die Verbindung auf Reibschluss basiert. Aufgrund des Reibschlusses ist es nicht möglich das Produktbehältnis in distale Richtung zu verschieben. Eine in distale Richtung auf das Produktbehältnis wirkende Kraft kann über den Reibschluss auf das Eingriffsglied und von dem Eingriffsglied auf den Produktbehältnishalter abgeleitet werden.

In einer noch weiteren Ausführungsform kann an dem Aufbewahrungsabschnitt, insbesondere an dessen zylindrischer Außenfläche, mindestens eine Vertiefung vorgesehen sein, in welche das Eingriffsglied eingreifen kann, so dass ein vorwiegend formschlüssiger Eingriff gebildet wird, der je nach Ausgestaltung der mindestens einen Vertiefung auch eine kraft- bzw. reibschlüssige Wirkkomponente haben kann.

Nach der Erfindung weist der Produktbehältnishalter ein Sicherungsmittel auf, das relativ zu dem Eingriffsglied aus einer Ausgangsposition in eine Sicherungsposition bewegbar ist. In der Sicherungsposition sichert das Sicherungsmittel den Eingriff des Eingriffsglieds dadurch, dass es die Bewegbarkeit des Eingriffsglieds einschränkt. Bevorzugt wird das Eingriffsglied in seiner Bewegbarkeit so eingeschränkt, dass es nicht mehr aus dem Eingriff mit dem Produktbehältnis gebracht werden kann. Das Sicherungsmittel kann auch dazu dienen, dass es das Eingriffsglied in den Eingriff mit dem Produktbehältnis spannt. Insbesondere wird mit dem Sicherungsmittel die Bewegbarkeit des Eingriffsglieds quer und zumindest weg von der Längsachse des Produktbehältnishalters blockiert, d.h. insbesondere, dass das Eingriffsglied nur durch Zerstörung des Sicherungsmittels weg von der Längsachse des Produktbehältnishalters bewegbar ist. Somit wird eine sichere Aufnahme des Produktbehältnisses in dem Produktbehältnishalter erreicht.

In der Ausgangsposition des Sicherungsmittels kann das Eingriffsglied in und aus den Eingriffen mit dem Produktbehältnis bewegt werden. Die Bewegung kann per Hand, durch ein Getriebe oder in bevorzugten Ausführungsformen durch ein federnd angeordnetes Eingriffsglied erzielt werden. Zum Beispiel kann die Federkraft das Eingriffsglied in Richtung des Eingriffs mit dem Produktbehältnis drücken.

Beim Einführen des Produktbehältnisses in den Produktbehältnishalter über eine proximale Öffnung des Produktbehältnishalters, wie es allgemein bevorzugt wird, kann das Eingriffsmittel in einen Eingriff mit dem Produktbehältnis gelangen, wie z.B. rasten oder angreifen, wenn das Produktbehältnis vollständig eingesetzt wurde. Zum Beispiel kann das federnd gelagerte Eingriffsglied auch zulassen, dass ein Produktbehältnis eingesetzt wird, das an seinem distalen Ende einen größeren Durchmesser aufweist als der Aufbewahrungsabschnitt und insbesondere auch die Stelle, an der das Eingriffsmittel bestimmungsgemäß in einen Eingriff mit dem Produktbehältnis kommen soll. Solch ein Abschnitt größeren Durchmessers kann zum Beispiel durch eine Nadelschutzkappe gebildet werden, die z. B. aus Gummi oder einem Hartkunststoff oder aus einer Kombination daraus sein kann. Die Nadelschutzkappe ist bereits bei der Auslieferung des mit Produkt gefüllten Produktbehältnisses am Produktbehältnis bzw. schützend über der Nadel angeordnet, um die Sterilität der Nadel zu wahren. Die Nadel darf oder kann zur Montage der Injektionsvorrichtung nicht abgenommen werden, da anderenfalls die Sterilität der Nadel nicht mehr gegeben ist. Die Erfindung erlaubt es das Produktbehältnis mitsamt Nadelschutzkappe in den Produktbehältnishalter einzuführen.

In der Ausgangsposition lässt das Sicherungsmittel die Bewegung des Eingriffsglieds zu, während es in der Sicherungsposition die Bewegung des Eingriffsglieds nicht mehr zulässt oder zumindest einschränkt. Das Sicherungsmittel befindet sich beim Einsetzen des Produktbehältnisses zweckmäßigerweise in der Ausgangsposition und wird anschließend in die Sicherungsposition bewegt, so dass das Produktbehältnis zumindest gegen eine Bewegung in distaler Richtung gesperrt ist.

In bevorzugten Ausführungsformen ist das Eingriffsglied flexibel bewegbar, d.h. das Eingriffsglied kann selber flexibel oder flexibel gelagert sein.

In bevorzugten Ausführungen kann der Produktbehältnishalter mindestens ein Führungsglied, vorzugsweise zwei, drei oder noch mehr Führungsglieder aufweisen, mit dem das Produktbehältnis seitlich führbar ist. Das eingesetzte Produktbehältnis wird von dem mindestens einen Führungsglied seitlich vorzugsweise so geführt, dass es kaum oder nur wenig seitliches Spiel hat. Das mindestens eine Führungsglied kann elastisch oder federnd nachgiebig sein oder elastisch bzw. federnd angeordnet sein, wobei das Eingriffsglied insbesondere an dem mindestens einen Führungsglied angeordnet sein kann. Je Führungsglied kann z. B. ein Eingriffsglied vorgesehen sein. Das oder die Führungsglieder können eine längliche Form aufweisen, wobei z.B. die Führungsglieder an ihren proximalen Enden miteinander, insbesondere einstückig verbunden sein können. Beispielsweise kann ein im Querschnitt ringförmiges Element die Führungsglieder miteinander verbinden. Somit können die Führungsglieder einstückig gebildet sein.

Alternativ oder zusätzlich kann der Produktbehältnishalter mehrere über den Umfang verteilte und voneinander getrennte Führungsglieder aufweisen. Die Führungsglieder können entweder in sich elastisch sein oder an ihren Befestigungsstellen mit dem Element, das die Führungsglieder miteinander verbindet, gelenkig oder elastisch gelagert sein. In besonders bevorzugten Ausführungen sind zwei Führungsglieder vorgesehen, welche am Umfang gegenüberliegend angeordnet sind, wobei zwischen den beiden Führungsgliedern bevorzugt eine Aussparung gebildet wird, durch die in das Innere des Produktbehältnishalters und bei einem eingelegten Produktbehältnis dessen Produkt gesehen werden kann. Die Aussparung kann in etwa eine solch axiale Länge aufweisen, wie der Kolben innerhalb des Produktbehältnisses verschiebbar ist.

Beispielsweise kann das Sicherungsmittel das Eingriffsglied umgeben, insbesondere einen Durchgang für das Eingriffsglied und/oder das Produktbehältnis bilden. Zum Beispiel kann das Eingriffsglied zwischen Produktbehältnis und Sicherungsmittel angeordnet sein, wenn sich das Sicherungsmittel in der Sicherungsposition befindet. Beispielsweise kann das Sicherungsmittel ein im Querschnitt ringförmiges Element sein, das das Produktbehältnis und das oder die Eingriffsglieder umgibt. Das zum Beispiel als Ring ausgestaltete Sicherungsmittel kann zum Beispiel eine entlang der Längsachse gemessene Länge aufweisen, die geringer ist als der Durchmesser des Rings. Bevorzugt ist die entlang der Längsrichtung gemessene Länge des Rings maximal viermal so groß wie der Durchmesser des Rings. Der Ring sollte allgemein so bemessen sein, dass das Produktbehältnis durch die Aussparung zwischen den Führungsgliedern noch erblickbar ist, wenn sich der Ring in seiner Sicherungsposition befindet. Allgemein kann daher gesagt werden, dass das Sicherungsmittel axial so bemessen ist, dass bei einem eingesetzten Produktbehältnis der Inhalt des Produktbehältnisses zum größten Teil bevorzugt vollständig erblickbar ist. Alternativ oder zusätzlich kann das Sicherungsmittel so ausgestaltet sein, dass sein proximales Ende in der Sicherungsposition maximal den Kolben des durch Ausschüttung vollständig entleerten Produktbehältnisses überdeckt. Das proximal hinter dem Kolben befindliche restliche Volumen des Produktbehältnisses kann dadurch vom Verwender noch erblickt werden.

Alternativ oder zusätzlich kann das Sicherungsmittel, insbesondere der Ring, im Wesentlichen unflexibel sein, d.h. deutlich weniger flexibel sein als die Anordnung der Eingriffsglieder oder des Eingriffsglieds.

Das Sicherungsmittel kann in seiner Sicherungsposition mit einem Verbindungsmittel relativ zu dem Eingriffsglied entlang der Längsachse unverschiebbar angeordnet sein. Insbesondere wird das Sicherungsmittel in der Sicherungsposition gegen eine Bewegung entlang der Längsachse gesperrt. Vorzugsweise wird das Sicherungsmittel auch gegen eine Verdrehung relativ zum Produktbehältnishalter, bzw. dem mindestens einen Eingriffsglied oder dem mindestens einem Führungsglied gesperrt. Dadurch kann sichergestellt werden, dass sich das Sicherungsmittel nicht versehentlich aus der Sicherungsposition bewegen kann. Das Verbindungsmittel basiert vorzugsweise auf einem Fonn- und/oder Kraftschluss. Zum Beispiel kann das Sicherungsmittel in der Sicherungsposition geklemmt sein, wobei es sich um eine kraftschlüssige Verbindung handelt. Besonders bevorzugt ist das Verbindungsmittel eine Rast- oder Schnappverbindung. Hierzu kann eines aus Sicherungsmittel und Eingriffs- bzw. Führungsglied eine Aussparung und das andere aus Sicherungsmittel und Eingriffs- bzw. Führungsglied einen Nocken aufweisen, wobei in der Sicherungsposition Nocken und Aussparung ineinander greifen und somit eine formschlüssige Verbindung bilden. Bevorzugt wird, dass das Verbindungsmittel, gemessen entlang der Längsachse, im Bereich des Eingriffsglieds angebracht ist. Ebenso ist es bevorzugt, dass das Sicherungsmittel im Bereich des Eingriffsglieds bzw. über dem Eingriffsglied angeordnet ist, sofern es sich in der Sicherungsposition befindet. Der Teil des Verbindungsmittels, der durch das Sicherungsmittel gebildet wird, kann sich in etwa in der Mitte zwischen dem distalen und proximalen Ende des Sicherungsmittels befinden.

In bevorzugten Ausführungsformen weist der Produktbehältnishalter eine Gesamtlänge auf, die in der Ausgangsposition größer ist als in der Sicherungsposition des Sicherungsmittels. Beispielsweise kann in der Sicherungsposition der Produktbehältnishalter um etwa die axiale Länge des Sicherungsmittels verkürzt sein, muss es aber nicht. Alternativ oder zusätzlich kann in der Ausgangsposition des Sicherungsmittels das Sicherungsmittel das distale Ende des Produktbehältnishalters bilden. Zum Beispiel kann das Sicherungsmittel distal des Eingriffsglieds und/oder des mindestens einen Führungsglieds angeordnet sein. Zum Beispiel ist das Sicherungsmittel an dem distalen Ende des mindestens einen Führungsglieds angeordnet.

In bevorzugten Ausführungen kann das Sicherungsmittel in seiner Ausgangsposition, in der es in Bezug auf das Eingriffsglied so angeordnet ist, dass das Eingriffsglied z.B. flexibel bewegbar ist, kraft-, form- oder stoffschlüssig an dein Produktbehältnishalter, insbesondere an einem Führungsglied gebildet sein. Beispielsweise kann das Sicherungsmittel in der Ausgangsposition in einer Klemmverbindung sein, so dass es kraftschlüssig an dem Produktbehältnishalter angeordnet ist. Beispielsweise kann das Sicherungsmittel mittels einer Kraft- oder Schnappverbindung an den Produktbehältnishalter angeordnet sein, wobei es sich um eine formschlüssige Verbindung handelt. Beispielsweise kann das Sicherungsmittel in seiner Ausgangsposition mit dem Produktbehältnishalter verklebt, verschweißt oder einstückig gebildet sein, so dass das Sicherungsmittel in seiner Ausgangsposition stoffschlüssig an den Produktbehältnishalter angeordnet ist. Bevorzugt wird, dass für die Bewegung des Sicherungsmittels aus seiner Ausgangsposition ein initialer Kraftaufwand erforderlich ist, der die Verbindung des Sicherungsmittels mit dem Produktbehältnishalter löst. Vorteilhaft wird hierdurch sichergestellt, dass das Sicherungsmittel in seiner Ausgangsposition bei der Handhabung des Produktbehältnishalters als eine separat handhabbare Einheit sich nicht vom Produktbehältnishalter löst bzw. richtig positioniert bleibt. Dies erleichtert die Verarbeitbarkeit des Produktbehältnishalters bzw. dessen Montage erheblich. Somit kann durch Aufbringen einer hinreichend großen Kraft zwischen Sicherungsmittel und dem restlichen Produktbehältnishalter die Verbindung erst dann gelöst werden, wenn dies auch wirklich gewollt ist.

In einer bevorzugten Ausführung kann das Sicherungsmittel über mindestens einen Steg an den restlichen Produktbehältnishalter, insbesondere an mindestens einen Führungsglied befestigt sein, wobei der Steg als Sollbruchstelle dient und bei der Bewegung des Sicherungsmittels in die Sicherungsposition oder aus der Ausgangsposition brechbar ist. Die Stege sind ein weiteres Beispiel für eine stoffschlüssige Verbindung, welche das Sicherungsmittel in seiner Ausgangsposition hält. Beispielsweise können mehrere Stege vorgesehen sein, wie zum Beispiel je Führungsglied zwei oder drei Stege, die die Führungsglieder mit dem Sicherungsmittel verbinden. Natürlich ist die Verbindung mit einem Steg je Führungsglied auch möglich.

Besonders bevorzugt ist, dass der Produktbehältnishalter d.h. auch einschließlich Sicherungsmittel einstückig mit einem Spritzgussverfahren hergestellt ist. Diese Ausführung ist von besonderem Vorteil, weil das Sicherungsmittel zum restlichen Produktbehältnishalter bereits beim Herstellen in der Spritzgussmaschine richtig positioniert ist. Dadurch verringern sich der Montageaufwand und die Kosten, da man lediglich nur ein einziges Teil handhaben muss und nicht mehrere.

Der beschriebene Produktbehältnishalter bildet mit dem beschriebenen Produktbehältnis, insbesondere der Spritze eine Baugruppe. Es wird bevorzugt, dass das Eingriffsglied entlang der Längsachse so angeordnet ist, dass es an das Produktbehältnis angreift, insbesondere an die von dem Produktbehältnis gebildete Schulter, die distal des Aufbewahrungsabschnitts, in dem der Kolben geführt ist, gebildet ist. Von Vorteil ist hierbei, wenn die axiale Position des Eingriffsglieds so ausgewählt ist, dass der Flansch des Produktbehältnisses kontaktfrei mit dem Produktbehältnishalter bleibt.

Ferner ist es bevorzugt, dass das Produktbehältnis eine Nadel aufweist, die mit einer Nadelschutzkappe abgedeckt ist, wobei das Eingriffsglied entlang der Längsachse an so einer Position angeordnet ist, dass es in einem Bereich zwischen Nadelschutzkappe und Aufbewahrungsabschnitt eingreift und der Flansch am Ende des Produktbehältnisses dennoch kontaktfrei zum Produktbehältnishalter bleibt.

Die Erfindung betrifft ferner ein Verfahren zur Montage einer Injektionsvorrichtung, insbesondere eines Autoinjektors. Das Verfahren umfasst folgende Schritte:
- Bereitstellen eines Produktbehältnisses, insbesondere einer Spritze, das ein Aufbewahrungsabschnitt zur Aufnahme eines Medikaments, eine Nadel an einem Ende des Aufbewahrungsabschnitts und einen im Aufbewahrungsabschnitt axial bewegbaren Kolben aufweist,
- Bereitstellen eines Produktbehältnishalters, insbesondere wie er hierin beschrieben wird, der ein Eingriffsglied für das Produktbehältnis aufweist,
- Zusammenfügen des Produktbehältnisses und des Produktbehältnishalters, wobei ein quer zu der Längsachse des Produktbehältnishalters, insbesondere flexibel bewegbar angeordnetes Eingriffsglied so an das Produktbehältnis angreift, dass eine Bewegung des Produktbehältnisses relativ zu dem Produktbehältnishalter in distale Richtung blockiert wird, wobei ein Sicherungsmittel relativ zu dem Eingriffsglied in eine Sicherungsposition bewegt wird, in der es den Eingriff des Eingriffsglieds dadurch sichert, dass es die flexible Bewegbarkeit des Eingriffsglieds einschränkt.

In einer ersten möglichen Ausführungsform ist es bevorzugt, dass zuerst das Produktbehältnis in den Produktbehältnishalter eingeführt und das Sicherungsmittel in die Sicherungsposition bewegt werden und anschließend die so gebildete Kombination aus Produktbehältnis und Produktbehältnishalter in eine erste Baugruppe eingeführt wird, die ein vorderes Gehäuse, insbesondere ein Auslöseelement für die Injektionsvorrichtung und vorzugsweise eine an das Auslöseelement angepasste Abziehkappe aufweist. Das Auslöseelement dient bei einem Autoinjektor bevorzugt dazu, an die Injektionsstelle angesetzt zu werden, wobei durch Andrücken des Auslöseelements an die Injektionsstelle die Verabreichung auslösbar ist. Es kann vorteilhaft sichergestellt werden, dass das Produktbehältnis mit dem Produktbehältnishalter außerhalb der ersten Baugruppe zusammengefügt und mit dem Sicherungsmittel gesichert werden kann.

In einer alternativen Ausführungsform ist es bevorzugt, dass zuerst der Produktbehältnishalter in die erste Baugruppe eingeführt wird und anschließend das Produktbehältnis in den Produktbehältnishalter eingeführt und das Sicherungsmittel in die Sicherungsposition bewegt werden. Vorzugsweise stützt sich das Sicherungsmittel an dem vorderen Gehäuse bzw. dem Auslöseelement oder an der Abziehkappe ab. Beim Einführen des Produktbehältnisses wird, zum Beispiel durch Ausübung einer Kraft auf dem Flansch, eine so hohe Kraft zwischen dem Sicherungsmittel und dem restlichen Produktbehältnishalter ausgeübt, dass die kraft-, form - oder stoffschlüssige Verbindung zwischen Produktbehältnishalter und Sicherungsmittel gelöst wird, so dass sich der restliche Produktbehältnishalter zusammen mit dem Produktbehältnis relativ zu dem Auslöseelement, der Abziehkappe und/oder dem Sicherungsmittel bewegt, bis das Sicherungsmittel die Sicherungsposition auf dem Produktbehältnishalter eingenommen hat.

Allgemein wird bevorzugt, dass bei der Bewegung des Sicherungsmittels in die Sicherungsposition einer Sollbruchstelle zwischen Sicherungsmittel und Produktbehältnishalter gelöst wird.

Ein weiterer bevorzugter Verfahrensschnitt ist, dass das Auslöseelement und der Produktbehältnishalter insbesondere rotatorisch und/oder translatorisch so zueinander positioniert werden, dass ein Sichtfenster, insbesondere eine Aussparung des Auslöseelements und ein Sichtfenster, insbesondere eine Aussparung des Produktbehältnishalters, die Sicht auf das Produktbehältnis frei geben. Wenn die erste Baugruppe in einem in ein Injektionsgerät eingefügten Zustand von einem Gehäuse umgeben wird, wird bevorzugt, dass auch das Gehäuse der Injektionsvorrichtung eine Aussparung oder ein Fenster aufweist, die oder das so zu den Sichtfenstern des Auslöseelements und des Produktbehältnishalters, insbesondere rotatorisch und translatorisch positioniert ist, dass die Sicht auf das Produktbehältnis freigegeben ist.

Bevorzugt wird eine zweite Baugruppe bereitgestellt, die den Antriebsmechanismus der Injektionsvorrichtung enthält und mit der ersten Baugruppe, die mit Produktbehältnis und Produktbehältnishalter zusammengefügt wurde, verbunden wird. Vorzugsweise wird die erste Baugruppe über eine Öffnung am distalen Ende eines vorzugsweise zylindrischen Gehäuses der Injektionsvorrichtung in die Injektionsvorrichtung eingeführt. Die erste Baugruppe verriegelt dann mit Elementen in der Injektionsvorrichtung.

Die Erfindung wurde anhand von mehreren Ausführungsbeispielen beschrieben. Im Folgenden wird eine bevorzugte Ausführungsform der Erfindung anhand von Figuren beschrieben. Die dabei offenbarten Merkmale bilden allein oder in Kombination mit den oben genannten Ausführungsbeispielen die Erfindung vorteilhaft weiter. Es zeigen:
- Figur 1a: eine Schnittdarstellung eines Produktbehältnishalters mit einem Sicherungselement in der Ausgangsposition,
- Figur 1b: den Produktbehältnishalter aus Figur 1a in einer um 90° um die Längsachse gedrehten Schnittdarstellung,
- Figur 1c: den Produktbehältnishalter aus Figur 1a in einer dreidimensionalen Ansicht,
- Figur 2a: eine Schnittdarstellung des Produktbehältnishalters aus Figur 1a mit einem Sicherungsmittel in der Sicherungsposition,
- Figur 2b: den Produktbehältnishalter aus Figur 2a in einer um 90° um die Längsachse gedrehten Schnittdarstellung,
- Figur 2c: den Produktbehältnishalter aus Figur 2a in einer dreidimensionalen Ansicht,
- Figur 3: eine Explosionsdarstellung des Produktbehältnishalters aus Figur 1a zusammen mit einem Produktbehältnis und einer ersten Baugruppe,
- Figur 4a: eine Schnittdarstellung der Teile aus Figur 3a in einem zusammengefügten Zustand und einem Sicherungsglied in der Ausgangsposition,
- Figur 4b: die zusammengefügten Teile aus Figur 4a in einer um 90° um die Längsachse gedrehten Schnittdarstellung,
- Figur 5a: die zusammengefügten Teile aus Figur 4a in einer Schnittdarstellung und mit einem Sicherungsmittel in der Sicherungsposition, und
- Figur 5b: die zusammengefügten Teile aus Figur 5a in einer um 90° um die Längsachse gedrehten Schnittdarstellung.

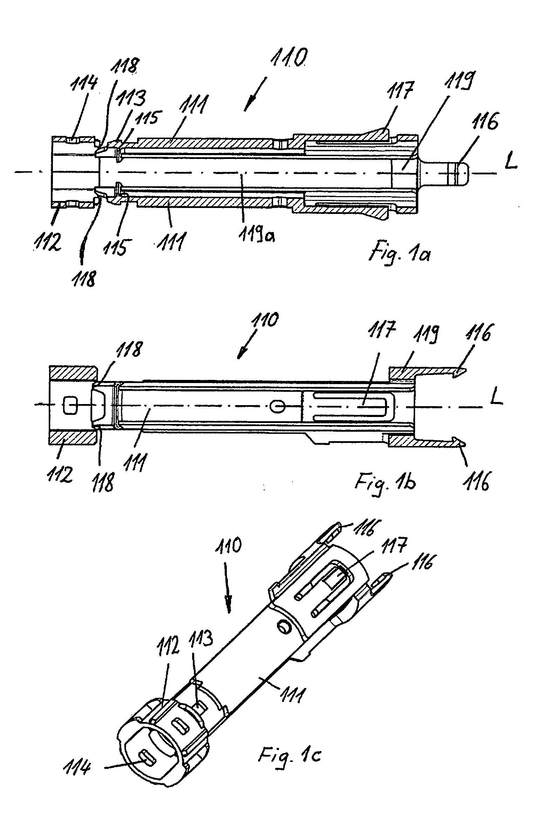

Alle Figuren, insbesondere die Figuren 1a, 1b und 1c zeigen einen Produktbehältnishalter 110, der mit einem Spritzgussverfahren einteilig hergestellt ist. Der Produktbehältnishalter 110 ist aus einem geeigneten Kunststoff geformt und weist einen Verbinder 119 oder eine Basis 119 auf, der oder die im Querschnitt ringförmig ist und genügend Platz bietet, dass ein Produktbehältnis über das proximale Ende in den Produktbehältnishalter 110 einführbar ist. An der Basis 119 sind zwei Befestigungsglieder 116 angeordnet, die sich in proximale Richtung erstrecken und am Ende hakenförmig ausgestaltet sind. Die Befestigungsglieder 116 sind bei der Montage des Produktbehältnishalters 110 in eine Injektionsvorrichtung beispielsweise mit einer funktionshülse verbindbar, die den Produktbehältnishalter 110 in der fertig montierten Injektionsvorrichtung zum Einstechen einer Nadel nach distal verschieben und nach einer erfolgten Verabreichung des Produkts auch wieder zurückziehen kann. Eine geeignete Funktionshülse und ein ebenso für die Erfindung geeignetes Autoinjektionsgerät wird in der DE 10 2007 013 836 A1 beschrieben. Die vorliegende Erfindung lässt sich besonders gut in die darin gezeigte Injektionsvorrichtung integrieren. Die DE 10 2007 013 836 A1 bezeichnet die Funktionshülse mit der Bezugsziffer 8.

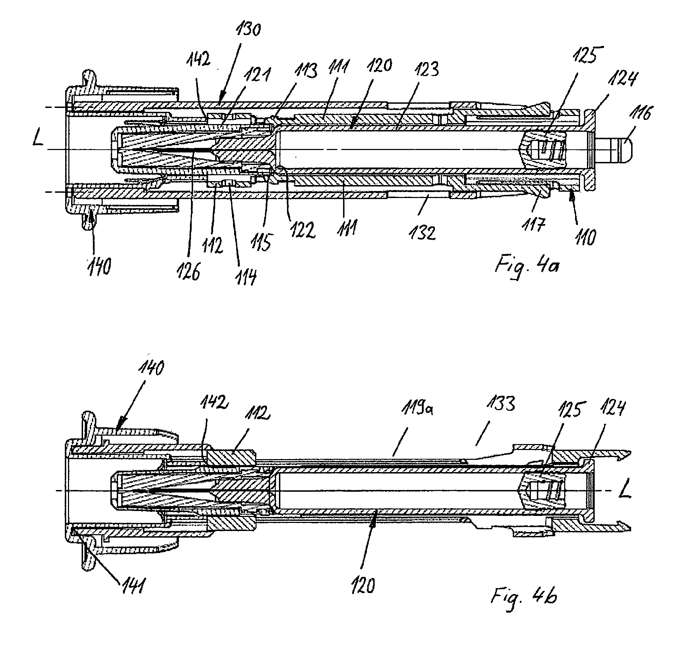

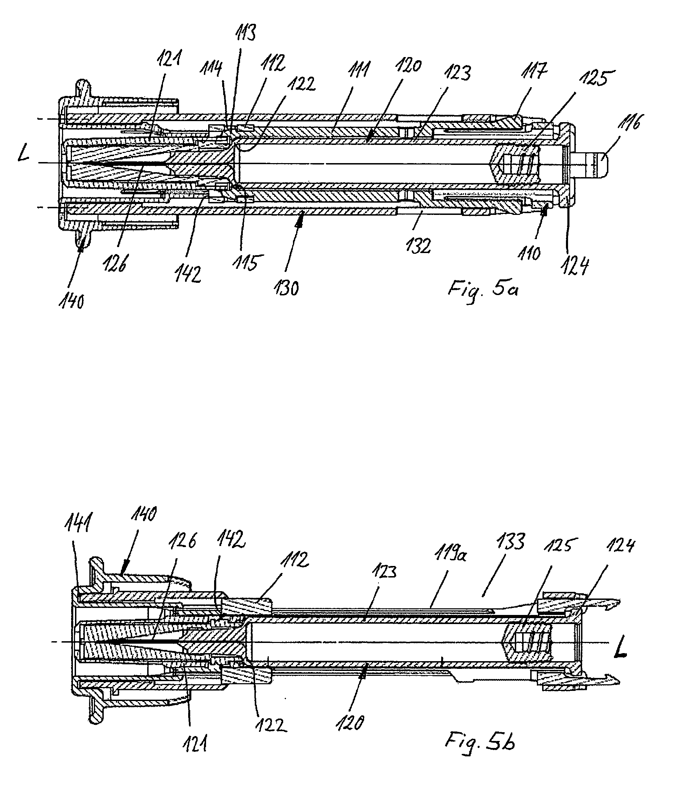

Von der Basis 119 erstrecken sich in distale Richtung zwei am Umfang gegenüberliegend ungeordnete Führungsglieder 111. Die Führungsglieder 111 dienen zum einen dazu, das in den Produktbehältnishalter 110 einsetzbare Produktbehältnis 120 seitlich einzufassen bzw. zu führen, so dass relativ wenig Spiel zwischen dem Produktbehältnis 120 und den Führungsgliedern 111 besteht. Das Produktbehältnis 120 ist am besten in den Figuren 4a, 4b und 5a, 5b erkennbar.

Der Produktbehältnishalter 110, insbesondere die Führungsglieder 111 bilden außerdem Schaltnocken 117, die federnd an einem Arm angeordnet sind und vom Umfang des Produktbehältnishalters 110 abragen. Die Schaltnocken 117 gehören zu einer Mechanik zum Rückzug des Produktbehältnishalters 110 nach erfolgter Produktverabreichung. Die Funktion der Schaltnocken 117 wird in der DE 10 2007 013 836 A1 beschrieben, auf die hiermit Bezug genommen wird, und die darin mit dem Bezugszeichen 17 bezeichnet sind.

Die Führungsglieder 111 weisen ferner jeweils ein oder mehrere Eingriffsglieder 115 auf, die an der zur Längsachse L hinweisenden Innenfläche der Führungsglieder 111 radial nach innen ragen. Die Eingriffsglieder 115 greifen formschlüssig an eine Schulter 122 des Produktbehältnisses 120 (Figur 4a) ein, um eine Reaktionsfläche zu bilden, die verhindert, dass das Produktbehältnis 120 relativ zu dem Produktbehältnishalter 110 in distale Richtung verschiebbar ist, wenn die Eingriffsglieder 115 in die Schulter 122 eingreifen. Die in proximale Richtung weisende Reaktionsfläche der Eingriffsglieder 115 ist entsprechend der Neigung der Schulter 122 angepasst. Eine auf das Produktbehältnis 120 in distale Richtung ausgeübte Kraft kann somit statt über den Flansch 124 über die Schulter 122 und die Eingriffsglieder 115 an den Produktbehältnishalter 110 abgeleitet werden.

Das distale Ende des Produktbehältnishalters 110 wird durch ein Sicherungsmittel 112 gebildet, das einen ringförmigen Querschnitt aufweist und mit den Führungsmitteln 111 jeweils mit mindestens einem Steg 118 verbunden ist. Die Stege 118 sind ebenfalls bei der Herstellung des Produktbehältnishalters 110 im Spritzgussverfahren hergestellt. Dies erlaubt eine einfache Handhabbarkeit des Produktbehältnishalters 110, da alle seine Teile bereits zueinander positioniert sind, wenn er aus dem Spritzgusswerkzeug ausgestoßen wird.

Der Produktbehältnishalter 110 weist Nocken 113 auf, die gemessen entlang der Längsachse L in etwa im Bereich der Eingriffsglieder 115 angeordnet sind. Die Nocken 113 werden von den Führungsglieder 111 gebildet und ragen radial nach außen ab. Die Nocken 113 dienen zum Eingriff in Aussparungen 114, die an dem Sicherungsmittel 112 gebildet sind.

Wie z. B. in den Figuren 4a und 4b erkennbar ist, weist das Produktbehältnis eine Nadelschutzhülse 121 auf, die einen größeren Außendurchmesser aufweist als der Aufbewahrungsabschnitt 123 des Produktbehältnisses 120.

Beim Einführen des Produktbehältnisses 120 in den Produktbehältnishalter 110 wird die Nadelschutzkappe 121 an den Eingriffsgliedern 115 vorbei bewegt. Die Eingriffsglieder 115 bewegen sich quer zur Längsachse L des Produktbehältnisses 120 dergestalt, dass der Querschnitt den die Eingriffsglieder 115 bilden, aufgeweitet wird, so dass die Nadelschutzkappe 121 die Eingriffsglieder 115 passieren kann. Sobald die Nadelschutzkappe 121 die Eingriffsglieder 115 passiert hat, federn diese in ihre ursprüngliche Position zurück. Die federnde Anordnung wird insbesondere durch die Stege 118 und/oder die Führungsglieder 111 bzw. deren Anordnung an der Basis 119 erreicht.

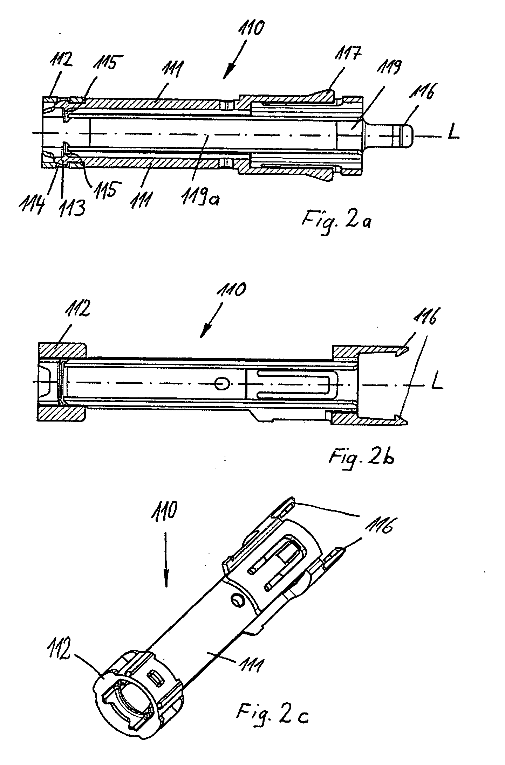

In den Figuren 2a, 2b und 2c wird der Produktbehältnishalter 110 aus den Figuren 1a, 1b und 1c dargestellt, allerdings mit einem in die Sicherungsposition verschobenen Sicherungsmittel 112. Wie besonders gut aus Figur 2a erkennbar ist, ist der Nocken 113 in die Aussparung 114 des Sicherungsmittels 112 eingerastet. Da das Sicherungsmittel 112 relativ unflexibel, insbesondere starr ist, hält das Sicherungsmittel 112 die Eingriffsglieder 115 in einer Position, in der sie in einem Eingriff mit dem Produktbehältnis 120 stehen. Das Sicherungsmittel 112 verhindert somit eine Bewegung der Eingriffsglieder 115 nach außen bzw. weg von der Längsachse L. Die Bewegung der Eingriffsglieder 115 ist somit eingeschränkt.

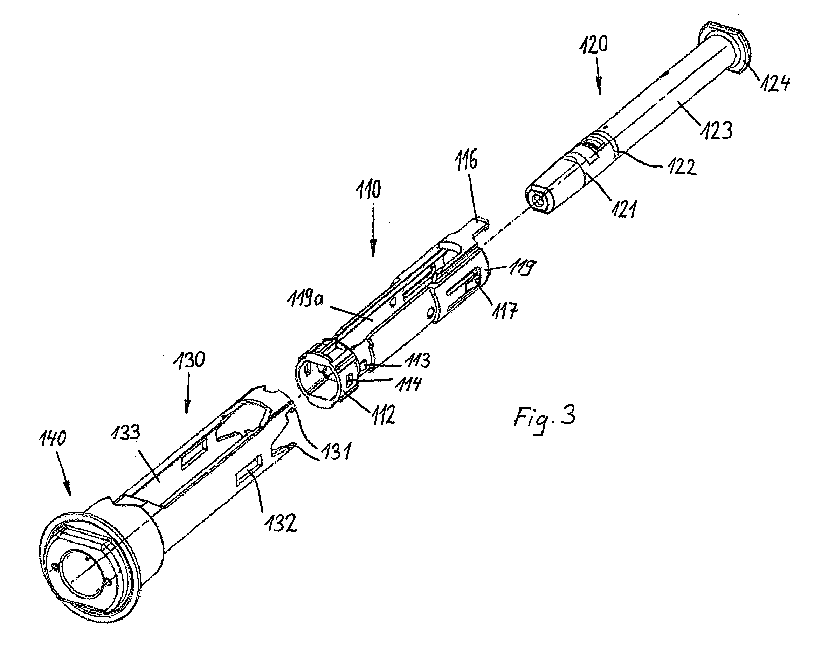

In Figur 3 wird der Produktbehältnishalter 110 zusammen mit dem Produktbehältnis 120 und einer ersten Baugruppe 130, 140, die aus einer Front- oder Betätigungshülse 130 und einer daran befestigten Abziehkappe 140 gebildet wird, in einer Explosionsdarstellung gezeigt. Die Darstellung eignet sich insbesondere dafür, um darzustellen, wie der Produktbehältnishalter 110 und das Produktbehältnis 120 in die erste Baugruppe 130, 140 integriert werden können.

In einer ersten, in den Figuren nicht dargestellten Möglichkeit, wird das Produktbehältnis 120 zunächst in den Produktbehältnishalter 110 eingeführt, bis die Eingriffsglieder 115 in die Schulter 122 eingreifen, nachdem die Nadelschutzkappe 121 die Eingriffsglieder 115 passiert hat. Anschließend wird zwischen dem Sicherungsmittel 112 und dem restlichen Produktbehältnishalter 110 eine Kraft ausgeübt, so dass die als Sollbruchstelle dienenden Stege 118 zerbrechen. Hierdurch sind Sicherungsmittel 112 und der Rest des Produktbehältnishalters 110 relativ zueinander bewegbar, so dass das Sicherungsmittel 112 in die Sicherungsposition verschoben und darin axial fixiert wird. Anschließend wird die Kombination aus Produktbehältnishalter 110 und Produktbehältnis 120 in die erste Baugruppe 130, 140 über das proximale Ende der Betätigungshülse 130 eingeführt. Schließlich wird die so gebildete Baugruppe über das distale Ende einer Injektionsvorrichtung in die Injektionsvorrichtung eingeführt, wobei die Haken 116 mit einer Funktionshülse und die Verbindungsmittel 131 mit einer Schalthülse, wie in der DE 10 2007 013 836 A1 offenbart, verrastet werden.

In einer anderen Möglichkeit der Montage wird zunächst der Produktbehältnishalter 110 in die Baugruppe 130, 140 eingeführt. Anschließend folgt das Produktbehältnis 120, das soweit eingeschoben wird, bis die Eingriffsglieder 115 in die Schulter 122 eingreifen. Diese Situation wird in den Figuren 4a und 4b dargestellt. Die Kappe 140 weist eine Stützfläche 142 auf, an der sich das Sicherungsmittel 112 mit seinem distalen Ende axial abstützt. Durch Aufbringen einer hinreichend großen Kraft auf das proximale Ende des Produktbehältnisses 120 bzw. des Produktbehältnishalters 110 zerbrechen die als Sollbruchstellen dienenden Stege 118, so dass Produktbehältnishalter 110 und Produktbehältnis 120 gemeinsam relativ zu dem Sicherungsmittel 112 axial bewegt werden, so lange bis die Nocken 113 in die Aussparungen 114 eingreifen, wie es in den Figuren 5a und 5b dargestellt ist.

Wie z. B. aus Figur 5a erkennbar ist, liegt der Flansch nach der Montage nicht an dem proximalen Ende des Produktbehältnishalters 110 auf. Somit kann ein Zerbrechen des Flansches 124 beim Auslösen der Injektionsvorrichtung verhindert werden.

Vor dem Auslösen der Injektionsvorrichtung wird die Abziehkappe 140 in distale Richtung abgezogen. Die Abziehkappe 140 greift mit einem Eingriffselement in die Nadelschutzkappe 121 ein und nimmt diese bei der Abziehbewegung mit, so dass die Abziehkappe 140 mitsamt Nadelschutzkappe 121 abgezogen werden können. Produktbehältnishalter 110 und Produktbehältnis 120 werden nicht mit herausgezogen, da der Produktbehältnishalter 110 mit den Befestigungsgliedern 116 axial fest an der Mechanik der Injektionsvorrichtung befestigt ist, die ihrerseits die Kraft in das Gehäuse ableitet, wie in der DE 10 2007 013 836 A1 beschrieben wird.

### Bezugszeichen:

- 110: Produktbehältnishalter, Spritzenhalter
- 111: Führungsglied
- 112: Sicherungsmittel
- 113: Nocken
- 114: Aussparung
- 115: Eingriffsglied
- 116: Befestigungsglied / Haken
- 117: Schaltnocken
- 118: Steg / Sollbruchstelle
- 119: Verbinder / Basis
- 119a: Sichtfenster
- 120: Produktbehältnis
- 121: Nadelschutzkappe
- 122: Schulter
- 123: Aufbewahrungsabschnitt
- 124: Flansch
- 125: Kolben
- 126: Nadel

- 130: Fronthülse/Betätigungshülse
- 131: Verbindungsmittel
- 132: Sperrfenster
- 133: Sichtfenster

- 140: Abziehkappe
- 141: Positionierelement
- 142: Stützfläche

- L: Längsachse

## Patentansprüche

1. Produktbehältnishalter zur Aufnahme eines Produktbehältnisses (120), wobei der Produktbehältnishalter (110) eine separat handhabbare Einheit und in eine Injektionsvorrichtung zur Verabreichung eines Produkts einsetzbar ist der Produktbehältnishalter umfassend:
a) ein quer zu der Längsachse (L) des Produktbehältnishalters (110) bewegbar angeordnetes Eingriffsgfied (115) für das Produktbehältnis (120), mit dem eine Bewegung des Produktbehältnisses (120) relativ zu dem Produktbehältnishalter (110) in distale Richtung blockierbar ist, und
b) ein Sicherungsmittel (112), das relativ zu dem Eingriffsglied (115) aus einer Ausgangsposition in eine Sicherungsposition bewegbar ist, **dadurch gekennzeichnet, dass**
das Sicherungsmittel (112) den Eingriff des Eingriffsglieds (115) guer und weg von der Längsachse (L) des Produktbehältnishalters (100) sichert, so dass es die Bewegbarkeit des Eingriffsglieds (115) einschränkt, wobei das Sicherungsmittel (112) einen Durchgang für das Eingriffsglied (115) bildet, und starr ist.

2. Produktbehältnishalter nach dem vorhergehenden Anspruch, **dadurch gekennzeichnet, dass** das Eingriffsglied (115) flexibel oder federnd bewegbar ist.

3. Produktbehältnishalter nach einem der vorhergehenden Ansprüche, **dadurch gekennzeichnet, dass** der Produktbehäftnishalter (110) mindestens ein Führungsglied (111) aufweist, mit dem das Produktbehältnis (120) seitlich führbar ist, wobei das mindestens eine Führungsglied (111) elastisch nachgiebig ist oder angeordnet ist, wobei das Eingriffsglied (115) insbesondere an dem mindestens einen Führungsglied (111) angeordnet ist.

4. Produktbehältnishalter nach einem der vorhergehenden Ansprüche, **dadurch gekennzeichnet, dass** mehrere über den Umfang verteilte und voneinander getrennte, insbesondere - längliche Führungsglieder (111) angeordnet sind, die an ihren proximalen Enden miteinander, insbesondere einstückig, und an ihren distalen Enden mit dem Sicherungsmittel (112) verbunden sind.

5. Produktbehältnishalter nach einem der vorhergehenden Ansprüche, **dadurch gekennzeichnet, dass** das Sicherungsmittel (112) in der Sicherungsposition mit einem Verbindungsmittel (113,114) relativ zu dem Eingriffsglied (115) axial unverschiebbar ist, wobei das Verbindungsmittel(113, 114) vorzugsweise auf einem Form- oder/und Kraftschluss basiert

6. Produktbehältnishalter nach Anspruch 5, **dadurch gekennzeichnet, dass** das Verbindungsmittel (113,114) gemessen entlang der Längsachse (L) im Bereich des Eingriffsglieds (115) angebracht ist.

7. Produktbehältnishalter nach einem der vorhergehenden Ansprüche, **dadurch gekennzeichnet, dass** der Produktbehältnishalter (110) eine Gesamtlänge aufweist, die in der Ausgangsposition grösser ist als in der Sicherungsposition des Sicherungsmittels (H 2), wobei in der Ausgangsposition des Sicherungsmittels (112) vorzugsweise das Sicherungsmittel (112) das distale Ende des Produktbehältnishalters (110) bildet.

8. Produktbehältnishalter nach einem der vorhergehenden Ansprüche, **dadurch gekennzeichnet, dass** das Sicherungsmittel (112) in seiner Ausgangsposition, in der es in Bezug auf das Eingriffsglied (115) so angeordnet ist, dass das Eingriffsglied (115) bewegbar ist, kraft-, form- oder stoffschlüssig an dem Produktbehältnishalter (110), insbesondere an mindestens einam Führungsglied (111) gebildet ist.

9. Produktbehältnishalter nach einem der vorhergehenden Ansprüche, dadurch gekennzeichet dass der Produktbehältnishalter (110) einschliesslich Sicherungsmittel (112) einstückig mit einem Spritzgussverfahren hergestelit ist

10. Produktbehältnishalter nach einem der vorhergehenden Ansprüche, **dadurch gekennzeichnet, dass** das Sichorungsmittel (112) über mindestens einen Steg (118) an dem Produktbehältnishalter (110) befestigt ist, wobei der Steg (118) als Sollbruchstelle dient und bei der Bewegung des Sicherungsrmittels (112) in die Sicherungsposition brechbar ist.

11. Baugruppe, umfassend den Produktbehältnishalter (110) nach einem der vorhergehenden Ansprüche und ein Produktbehältnis (120), insbesondere eine Spritze, wobei das Eingriffsglied (115) an das Produktbehältnis (120) angreift, insbesondere an eine von dem Produktbehältnis (110) gebildete Schulter (122), die distal eines Aufbewahrungsabschnitts (123), in dem ein Kolben (125) geführt ist, gebildet ist

12. Baugruppe nach dem vorhergehenden Anspruch, **dadurch gekennzeichnet, dass** das Produktbehältnis (120) eine Nadel (126) aufweist, die mit einer Nadelschutzkappe (121) abgedeckt ist, wobei das Eingriffsglied (115) in einem Bereich zwischen Nadelschutzkappe (121) und Aufbewahrungsabschnitt (123) eingreift.

13. Verfahren zur Montage einer injektionsvorrichtung, insbesondere einer Autoinjektionsvorrichtung, folgende Schritte umfassend:
a) Bereitstellen eines Produktbehältnisses (120), insbesondere einer Spritze, die einen Aufbewahrungsabschnitt (123) zur Aufnahme eines Medikaments, eine Nadel (126) an einem Ende des Aufbewahrungsabschnitts (123) und einen im Aufbewahrungsabschnitt (123) axial bewegbaren Kolben (125) aufweist
b) Bereitstellen eines Produktbehältnishalters (110) nach den Ansprüchen 1 bis 12, der ein Eingriffsglied (115) für das Produktbehältnis (120) aufweist,
c) Zusammenfügen des Produktbehältnisses (120) und des Produktbehältnishalters (110), wobei ein quer zu der Längsachse (L) des Produktbehältnishalters (110), insbesondere bewegbar angeordnetes Eingriffsglied (115) so an das Produktbehältnis (120) angreift, dass eine Bewegung des Produktbehältnisses (120) relativ zu dem Produktbehältnishalter (110) in distale Richtung blockiert wird, wobei
ein Sicherungsmittel (112) relativ zu dem Eingriffsglied (115) in eine Sicherungsposition bewegt wird, in der es den Eingriff des Eingriffsglieds (115) dadurch sichert, dass es die flexible Bewegbarkeit des Eingriftsglieds (115) einschränkt

14. Verfahren nach dem vorhergehenden Anspruch, **dadurch gekennzeichnet, dass** zuerst das Produktbehältnis (120) in den Produktbehältnishalter 110) eingeführt und das Sicherungsmittel (112) in die Sicherungsposition bewegt werden und anschliessend die so gebildete Kombination aus Produktbehältnis (120) und Produktbehältnishalter (110) in eine erste Baugruppe (130,140) eingeführt wird; die ein vorderes Gehäuse, insbesondere ein Auslöseelement (130) für die Injektionsvorrichtung, und vorzugsweise eine an das vordere Gehäuse bzw. das Auslöseelement (130) angepasste Abziehkappe (140) aufweist.

15. Verfahren nach Anspruch 14, **dadurch gekennzeichnet, dass** zuerst der Produktbehältnishalter (110) in eine erste Baugruppe (130, 140) eingeführt wird, die ein vorderes Gehäuse, insbesondere eine Auslöseelement (130) für die Injektionsvorrichtung und vorzugsweise eine an das Auslöseelement (130) angepasste Abziehkappe (140) aufweist, und anschliessend das Produktbehältnis (120) in den Produktbehältnishalter (110) eingeführt und das Sicherungsmittel (112) in die Sicherungsposition bewegt werden.

16. Verfahren nach einem der drei vorhergehenden;Anspräche, wobei bei der Bewegung des Sicherungsmittels (112) in die Sicherungsposition eine Sollbruchstelle (118) zwischen Sicherungsmittel (112) und Produktbehältnishalter (110) gelöst wird.

17. Verfahren nach einem der beiden vorhergehenden Ansprüche, wobei das Auslöseelement (130) und der Produktbehältnishalter (110) rotatorisch so zueinander positioniert werden, dass ein Sichtfenster (133), insbesondere eine Aussparung des Auslöseelements (130), und ein Sichtfenster (119a), insbesondere eine Aussparung des Produktbehältnishalters (110), die Sicht auf das Produktbehältnis (120) freigeben.

## Claims

1. A product container holder for accommodating a product container (120), wherein the product container holder (110) is a unit which can be handled separately and can be inserted into an injection device to administer a product, the product container holder comprising:
a) an engagement member (115) for the product container (120), that is arranged moveably transversely relative to the longitudinal axis (L) of the product container holder (110) and with which a movement of the product container (120) relative to the product container holder (110) in the distal direction can be blocked;
b) a securing means (112) which is movable relative to the engagement member (115) from an initial position into a securing position,
**characterised in that** the securing means (112) secures the engagement of the engagement member (115) transversely and away from the longitudinal axis (L) of the product container holder (110) so that it restricts the mobility of the engagement member (115), wherein the securing means (112) forms a passage for the engagement member (115) and is rigid.

2. A product container holder according to the preceding claim **characterised in that** the engagement member (115) is moveable flexibly or springily

3. A product container holder according to one of the preceding claims **characterised in that** the product container holder (110) has at least one guide member (111) with which the product container (120) can be laterally guided, wherein the at least one guide member (111) is or is arranged to be elastically yielding, wherein the engagement member (115) is arranged in particular on the at least one guide member (111).

4. A product container holder according to one of the preceding claims **characterised in that** there are a plurality of in particular elongate guide members (111) which are distributed over the periphery and separated from each other and are connected to each other, in particular integrally, at their proximal ends and to the securing means (120) at their distal ends.

5. A product container holder according to one of the preceding claims **characterised in that** the securing means (112) is axially immovable in the securing position with a connecting means (113, 114) relative to the engagement member (115), wherein the connecting means (113, 114) is preferably based on a positive fit and/or a force-locking fit.

6. A product container holder according to claim 5 **characterised in that** the connecting means (113, 114) as measured along the longitudinal axis (L) is attached in the region of the engagement member (115).

7. A product container holder according to one of the preceding claims **characterised in that** the product container holder (110) is of an overall length greater in the initial position than in the securing position of the securing means (112), wherein preferably the securing means (112) forms the distal end of the product container holder (110) in the initial position of the securing means (112).

8. A product container holder according to one of the preceding claims **characterised in that** the securing means (112) in its initial position in which it is arranged in relation to the engagement member (115) such that the engagement member (115) is movable is formed in a force-locking fit, a positive fit or material-locking fit on the product container holder (110), in particular on the at least one guide member (111).

9. A product container holder according to one of the preceding claims **characterised in that** the product container holder (110) including the securing means (120) is made in piece using an injection-moulding method.

10. A product container holder according to one of the preceding claims **characterised in that** the securing means (112) is fastened to the product container holder (110) by way of at least one limb (118), wherein the limb (118) serves as a desired-rupture location and can be ruptured when the securing means (112) moves into the securing position.

11. An assembly comprising the product container holder (110) according to one of the preceding claims and a product container (120), in particular a syringe, wherein the engagement member (115) engages the product container (120), in particular a shoulder (122) which is formed by the product container (110) and which is formed distally of a storage portion (123) in which a piston (125) is guided.

12. An assembly according to the preceding claim **characterised in that** the product container (120) comprises a needle (126)covered by a needle protective cap (121), wherein the engagement member (115) engages in a region between the needle protective cap (121) and the storage portion (123).

13. A method of assembling an injection device, in particular an automatic injection device, comprising the following steps:
a) providing a product container (120), in particular a syringe, comprising a storage portion (123) for accommodating a drug, a needle (126) at one end of the storage portion (123), and a piston (125) axially movable in the storage portion (123),
b) providing a product container holder (110) according to claims 1 to 12 comprising an engagement member (115) for the product container (120),
c) assembling the product container (120) and the product container holder (110), wherein an engagement member (115) which is in particular arranged moveably transversely relative to the longitudinal axis (L) of the product container holder (110) so engages the product container (120) that a movement of the product container (120) relative to the product container holder (110) in the distal direction is blocked, wherein a securing means (112) is moved relative to the engagement member (115) into a securing position in which it secures the engagement of the engagement member (115) by restricting the flexible mobility of the engagement member (115).

14. A method according to the preceding claim **characterised in that** the product container (120) is first inserted into the product container holder (110) and the securing means (112) moved into the securing position, and the combination of the product container (120) and the product container holder (110) formed **in that** way is then inserted into a first assembly (130, 140) which comprises a front housing, in particular a triggering element (130) for the injection device and preferably a pull-off cap (140) which is adapted to the front housing or triggering element (140) respectively.

15. A method according to claim 14 **characterised in that** the product container holder (110) is first inserted into a first assembly (130, 140) comprising a front housing, in particular a triggering element (130) for the injection device and preferably a pull-off cap (140) adapted to the triggering element (130), and the product container (120) is then inserted into the product container holder (110) and the securing means (112) moved into the securing position.

16. A method according to one of the three preceding claims wherein a desired-rupture location (118) between the securing means (112) and the product container holder (110) is released when the securing means (112) moves into the securing position.

17. A method according to one of the two preceding claims wherein the triggering element (130) and the product container holder (110) are rotationally positioned with respect to each other such that a viewing window (133), in particular an opening, of the triggering element (130) and a viewing window (119a), in particular an opening, of the product container holder (110) permit a view on to the product container (120).

## Revendications

1. Support de réservoir à produit pour la réception d'un réservoir à produit (120), sachant que le support de réservoir à produit (110) est une unité manipulable séparément et peut être inséré dans un dispositif d'injection pour l'administration d'un produit, le support de réservoir à produit comprenant :
a) un organe d'engagement (115) disposé de manière mobile transversalement à l'axe longitudinal (L) du support de réservoir à produit (110) pour le réservoir à produit (120), avec lequel un mouvement du réservoir à produit (120) par rapport au support de réservoir à produit (110) peut être bloqué dans le sens distal, et
b) un moyen d'arrêt (112) qui peut être déplacé par rapport à l'organe d'engagement (115) d'une position de départ à une position d'arrêt, **caractérisé en ce que**
le moyen d'arrêt (112) arrête l'engagement de l'organe d'engagement (115) transversalement et loin de l'axe longitudinal (L) du support de réservoir à produit (100), de sorte qu'il limite la mobilité de l'organe d'engagement (115), sachant que le moyen d'arrêt (112) forme un passage pour l'organe d'engagement (115) et est rigide.

2. Support de réservoir à produit selon la revendication précédente, **caractérisé en ce que** l'organe d'engagement (115) est mobile de manière flexible ou élastique.

3. Support de réservoir à produit selon l'une quelconque des revendications précédentes, **caractérisé en ce que** le support de réservoir à produit (110) présente au moins un organe de guidage (111), avec lequel le réservoir à produit (120) peut être guidé latéralement, sachant que l'au moins un organe de guidage (111) est flexible et élastique ou est disposé de manière flexible et élastique, sachant que l'organe d'engagement (115) est disposé en particulier sur l'au moins un organe de guidage (111).

4. Support de réservoir à produit selon l'une quelconque des revendications précédentes, **caractérisé en ce que** plusieurs organes de guidage en particulier oblongs, répartis sur la périphérie et séparés les uns des autres, sont disposés, lesquels sont reliés au niveau de leurs extrémités proximales les uns aux autres, en particulier d'un seul tenant et au niveau de leurs extrémités distales au moyen d'arrêt (112).

5. Support de réservoir à produit selon l'une quelconque des revendications précédentes, **caractérisé en ce que** le moyen d'arrêt (112) est immobile axialement dans la position d'arrêt avec un moyen de liaison (113, 114) par rapport à l'organe d'engagement (115), sachant que le moyen de liaison (113, 114) se base de préférence sur une liaison à force et/ou à complémentarité de formes.

6. Support de réservoir à produit selon la revendication 5, **caractérisé en ce que** le moyen de liaison (113, 114), mesuré le long de l'axe longitudinal (L), est monté dans la zone de l'organe d'engagement (115).

7. Support de réservoir à produit selon l'une quelconque des revendications précédentes, **caractérisé en ce que** le support de réservoir à produit (110) présente une longueur entière qui est supérieure dans la position de départ du moyen d'arrêt à celle dans la position d'arrêt du moyen d'arrêt (H2), sachant que dans la position de départ du moyen d'arrêt (112), le moyen d'arrêt (112) forme de préférence l'extrémité distale du support de réservoir à produit (110).

8. Support de réservoir à produit selon l'une quelconque des revendications précédentes, **caractérisé en ce que** le moyen d'arrêt (112) est formé dans sa position de départ, dans laquelle il est disposé par rapport à l'organe d'engagement (115) de sorte que l'organe d'engagement (115) soit mobile, à force, par complémentarité de formes ou par liaison de matière sur le support de réservoir à produit (110), en particulier sur au moins un organe de guidage (111).

9. Support de réservoir à produit selon l'une quelconque des revendications précédentes, **caractérisé en ce que** le support de réservoir à produit (110), y compris le moyen d'arrêt (112), est fabriqué d'un seul tenant dans un procédé de moulage par injection.

10. Support de réservoir à produit selon l'une quelconque des revendications précédentes, **caractérisé en ce que** le moyen d'arrêt (112) est fixé par au moins une nervure (118) sur le support de réservoir à produit (110), sachant que la nervure (118) sert de point destiné à la rupture et peut être rompue lors du mouvement du moyen d'arrêt (112) dans la position d'arrêt.

11. Sous-groupe comprenant le support de réservoir à produit (110) selon l'une quelconque des revendications précédentes, et un réservoir à produit (120), en particulier une seringue, sachant que l'organe d'engagement (115) agit sur le réservoir à produit (120), en particulier sur un épaulement (122) formé sur le réservoir à produit (120) de manière distale par rapport à une section de stockage (123) dans laquelle un piston (125) est guidé.

12. Sous-groupe selon la revendication précédente, **caractérisé en ce que** le réservoir à produit (120) présente une aiguille (126) qui est recouverte par un couvercle protecteur d'aiguille (121), sachant que l'organe d'engagement (115) s'engage dans une zone entre le couvercle protecteur d'aiguille (121) et la section de stockage (123).

13. Procédé de montage d'un dispositif d'injection, en particulier d'un dispositif d'autoinjection, comprenant les étapes suivantes :
a) mettre à disposition un réservoir à produit (120), en particulier une seringue qui présente une section de stockage (123) pour le logement d'un médicament, une aiguille (126) sur une extrémité de la section de stockage (123) et un piston (125) mobile axialement dans la section de stockage (123),
b) mettre à disposition un support de réservoir à produit (110) selon les revendications 1 à 12 qui présente un organe d'engagement (115) pour le réservoir de produit (120),
c) assembler le réservoir à produit (120) et le support de réservoir à produit (110), sachant qu'un organe d'engagement (115) disposé en particulier de manière mobile transversalement à l'axe longitudinal (L) du support de réservoir à produit (110) agit sur le réservoir à produit (120) de sorte qu'un mouvement du réservoir à produit (120) par rapport au support de réservoir à produit (110) soit bloqué dans le sens distal, sachant qu'un organe d'arrêt (112) est déplacé par rapport à l'organe d'engagement (115) dans une position d'arrêt, dans laquelle il arrête l'engagement de l'organe d'engagement (115) du fait qu'il limite la mobilité flexible de l'organe d'engagement (115).

14. Procédé selon la revendication précédente, **caractérisé en ce que** tout d'abord le réservoir à produit (120) est introduit dans le support de réservoir à produit (110) et le moyen d'arrêt (112) est déplacé dans la position d'arrêt et la combinaison de réservoir à produit (120) et de support de réservoir à produit (110) ainsi formée est ensuite introduite dans un premier sous-groupe (130, 140) qui présente un boîtier avant, en particulier un élément de déclenchement (130) pour le dispositif d'injection, et de préférence un capuchon détachable (140) adapté au boîtier avant ou à l'élément de déclenchement (130).

15. Procédé selon la revendication 14, **caractérisé en ce que** tout d'abord le support de réservoir à produit (110) est introduit dans un premier sous-groupe (130, 140) qui présente un boîtier avant, en particulier un élément de déclenchement (130) pour le dispositif d'injection, et de préférence un capuchon détachable (140) adapté à l'élément de déclenchement (130), et le réservoir à produit (120) est ensuite introduit dans le support de réservoir à produit (110) et le moyen d'arrêt (112) est déplacé dans la position d'arrêt.

16. Procédé selon l'une quelconque des trois revendications précédentes, dans lequel un point destiné à la rupture (118) est détaché entre le moyen d'arrêt (112) et le support de réservoir à produit (110) lors du mouvement du moyen d'arrêt (112) dans la position d'arrêt.

17. Procédé selon l'une quelconque des deux revendications précédentes, dans lequel l'élément de déclenchement (130) et le support de réservoir à produit (110) sont positionnés de manière rotative l'un par rapport à l'autre de sorte qu'un regard (133), en particulier un évidement de l'élément de déclenchement (130), et un regard (119a), en particulier un évidement du support de réservoir à produit (110), libèrent la vue sur le réservoir à produit (120).
